# EUROPEAN PATENT SPECIFICATION

(11) **EP 2 324 853 B1**
(45) Date of publication and mention of the grant of the patent: **26.08.2015**
(21) Application number: 09175876.3
(22) Date of filing: 13.11.2009
(51) Int. Cl.: A61K 31/155, A61K 45/06, A61K 38/26

(54) **Lixisenatide as add-on to metformin in the treatment of diabetes type 2**
Lixisenatid zusätzlich zu Metformin bei der Behandlung von Diabetes Typ 2
Lixisenatide en tant que traitement d'appoint à la metformine dans le diabète de type 2

(43) Date of publication of application: 25.05.2011
(73) Proprietor: Sanofi-Aventis Deutschland GmbH, 65929 Frankfurt am Main (DE)
(72) Inventor: Silvestre, Louise, 75013 Paris (FR); Sert-Langeron, Caroline, 75013 Paris (FR); Zhou, Tianyue, Bridgewater, NJ 08807 (US)
(74) Representative: Weickmann & Weickmann

(56) References cited:
- Gabriel S: "New Diabetes Compound AVE0010 Showed Clear Dose Response Results with one-a-Day Injection in Phase IIb Study" Press release Sanofi-aventis, ADA Annual Scientific Sessions 7 June 2008 (2008-06-07), pages 1-2, XP002572653 Retrieved from the Internet: URL:http://www.zealandpharma.com/pdf/Press -release-June-2008-Phase-II-data-on-AVE001 0.pdf> [retrieved on 2010-03-11]
- ROSENSTOCK J; RATNER R E; BOKA G: "Dose range effects of the new once daily GLP-1 receptor agonist AVE0010 added to metformin in type 2 diabetes" DIABETOLOGIA, vol. 51, no. Suppl. 1, September 2008 (2008-09), page S66, XP009130805 44TH ANNUAL MEETING OF THE EUROPEAN-ASSOCIATION-FOR-THE-STUDY-OF-DIAB ETES; ROME, ITALY; SEPTEMBER 08 -11, 2008 ISSN: 0012-186X
- CAMPAS C; CASTANER R: "AVE-0010 GLP-1 Receptor Agonist Treatment of Diabetes" DRUGS OF THE FUTURE, vol. 33, no. 10, October 2008 (2008-10), pages 838-840, XP002572655 ISSN: 0377-8282
- CHRISTENSEN M; KNOP F K; HOLST J J; VILSBOLL T: "Lixisenatide, a novel GLP-1 receptor agonist for the treatment of type 2 diabetes mellitus." IDRUGS : THE INVESTIGATIONAL DRUGS JOURNAL AUG 2009, vol. 12, no. 8, August 2009 (2009-08), pages 503-513, XP002572656 ISSN: 2040-3410
- RATNER R E; ROSENSTOCK J; BOKA G: "A dose-finding study of the new GLP-1 agonist AVE0010 in type 2 diabetes insufficiently controlled with metformin" DIABETES, vol. 57, no. Suppl. 1, June 2008 (2008-06) , page A129, XP009130802 68TH ANNUAL MEETING OF THE AMERICAN-DIABETES-ASSOCIATION; SAN FRANCISCO, CA, USA; JUNE 06 -10, 2008 ISSN: 0012-1797

## Description

Subject of the present invention is the treatment of diabetes type 2 with AVE0010 (lixisenatide) as add-on therapy to administration of metformin.

Gabriel (Press release Sanofi-aventis, 7 June 2008), Ratner (DIABETES, vol. 57, no. Suppl. 1, 2008, 68th Annual Meeting of the American-Diabetes-Association, Abstract no. 433-P) and Rosenstock (DIABETOLOGIA, vol. 51, Suppl. 1, 2008, p. S66) disclose that AVE0010 is well tolerated and significantly improves glycemic control in type 2 diabetes patients inadequately controlled with metformin alone.

DeFronzo (Diabetes Care 2005, vol. 28, no. 5, May 2005, pp. 1092-1100) describes effects of exendin-4 on glycemic control in metformin-treated patient with type 2 diabetes. Exendin-4 was generally well tolerated and reduced HbA1c in patients with type 2 diabetes failing to achieve glycemic control with metformin.

Metformin is a biguanide hypoglycemic agent used in the treatment of Type 2 diabetes mellitus not responding to dietary modification. Metformin improves glycemic control by improving insulin sensitivity. Metformin is usually administered orally. However, control diabetes mellitus type 2 in obese patients by metformin may be insufficient. Thus, in these patients, additional measures for controlling diabetes mellitus type 2 may be required.

A first aspect of the present invention is a combination for use in the treatment of diabetes mellitus type 2, the combination comprising
(a) desPro³⁶Exendin-4(1-39)-Lys₆-NH₂ or/and a pharmaceutically acceptable salt thereof,
(b) metformin or/and a pharmaceutically acceptable salt thereof,
wherein the subject to be treated is obese and has a body mass index of at least 30 kg/m² and has an age in the range of 18 to 50 years, and wherein the diabetes mellitus type 2 is not adequately controlled with metformin alone, and wherein desPro³⁶Exendin-4(1-39)-Lys₆-NH₂ or/and the pharmaceutically acceptable salt thereof is prepared for administration by one injection per day.

The compounds of (a) and (b) may be administered to a subject in need thereof, in an amount sufficient to induce a therapeutic effect.

The compound desPro³⁶Exendin-4(1-39)-Lys₆-NH₂ (AVE0010, lixisenatide) is a derivative of Exendin-4. AVE0010 is disclosed as SEQ ID NO:93 in WO 01/04156:
- SEQ ID NO: 1 AVE0010 (44 AS)
- SEQ ID NO: 2 Exendin-4 (39 AS)

Exendins are a group of peptides which can lower blood glucose concentration. The Exendin analogue AVE0010 is characterised by C-terminal truncation of the native Exendin-4 sequence. AVE0010 comprises six C-terminal lysine residues not present in Exendin-4.

In the context of the present invention, AVE0010 includes pharmaceutically acceptable salts thereof. The person skilled in the art knows pharmaceutically acceptable salts of AVE0010. A preferred pharmaceutically acceptable salt of AVE0010 employed in the present invention is acetate.

AVE0010 (desPro³⁶Exendin-4(1-39)-Lys₆-NH₂) or/and a pharmaceutically acceptable salt thereof may be administered by subcutaneous injection. Suitable injection devices, for instance the so-called "pens" comprising a cartridge comprising the active ingredient, and an injection needle, are known. AVE001 0 or/and a pharmaceutically acceptable salt thereof may be administered in a suitable amount, for instance in an amount in the range of 10 to 15 µg per dose or 15 to 20 µg per dose once a day (progressive titration from 10 to 15 and to 20 µg/day. 20 µg is the effective maintenance dose).

In the present invention, AVE0010 or/and a pharmaceutically acceptable salt thereof may be administered in a daily dose in the range of 10 to 15 µg or in the range of 15 to 20 µg once a day (progressive titration from 10 to 15 and to 20 µg/day. 20 µg is the effective maintenance dose). AVE0010 or/and a pharmaceutically acceptable salt thereof may be administered by one injection per day.

In the present invention, a liquid composition comprising desPro³⁶Exendin-4(1-39)-Lys₆-NH₂ or/and a pharmaceutically acceptable salt thereof may be employed. The skilled person knows liquid compositions of AVE001 0 suitable for parenteral administration. A liquid composition of the present invention may have an acidic or a physiologic pH. An acidic pH preferably is in the range of pH 1 - 6.8, pH 3.5 - 6.8, or pH 3.5 - 5. A physiologic pH preferably is in the range of pH 2.5 -8.5, pH 4.0 to 8.5, or pH 6.0 to 8.5. The pH may be adjusted by a pharmaceutically acceptable diluted acid (typically HCl) or pharmaceutically acceptable diluted base (typically NaOH). The preferred pH is in the range of pH 3,5 to 5,0.

The liquid composition may contain a buffer, such as a phosphate, a citrate, an acetate. Preferably, it can contain an acetate buffer, in quantities up to 5 µg/mL, up to 4 µg/mL or up to 2 µg/mL.

The liquid composition of the present invention may comprise a suitable preservative. A suitable preservative may be selected from phenol, m-cresol, benzyl alcohol and p-hydroxybenzoic acid ester. A preferred preservative is m-cresol.

The liquid composition of the present invention may comprise a tonicity agent. A suitable tonicity agent may be selected from glycerol, lactose, sorbitol, mannitol, glucose, NaCl, calcium or magnesium containing compounds such as CaCl₂. The concentration of glycerol, lactose, sorbitol, mannitol and glucose may be in the range of 100 - 250 mM. The concentration of NaCl may be up to 150 mM. A preferred tonicity agent is glycerol.

In addition, the liquid composition may contain L-methionin from 0,5 µg/mL to 20 µg/mL, preferably from 1 µg/mL to 5 µg/mL. Preferably, it contains L-methionin.

Metformin is the international non proprietary name of 1,1-dimethylbiguanide (CAS Number 657-24-9). In the present invention, the term "metformin" includes any pharmaceutically acceptable salt thereof.

In the present invention, metformin may be administered orally. The skilled person knows formulations of metformin suitable for treatment of diabetes type 2 by oral administration. Metformin may be administered in a dose of at least 1.0 g/day or at least 1.5 g/day. For oral administration, metformin may be formulated in a solid dosage form, such as a tablet or pill.

In the present invention, desPro³⁶Exendin-4(1-39)-Lys₆-NH₂ or/and a pharmaceutically acceptable salt is administered in an add-on therapy to administration of metformin.

In the present invention, the terms "add-on", "add-on treatment" and "add-on therapy" relate to treatment of diabetes mellitus type 2 with metformin and AVE0010. Metformin and AVE0010 may be administered within a time interval of 24 h. Metformin and AVE0010 each may be administered in a once-α-day-dosage. Metformin and AVE0010 may be administered by different administration routes. Metformin may be administered orally, and AVE0010 may be administered subcutaneously.

The subject to be treated with the combination of the present invention may have a HbA1c value in the range of 7 % to 10 %.

The subject to be treated with the combination of the present invention may be an adult subject.

Preferably, the diabetes type 2 is not adequately controlled by treatment with metformin alone with a dose of at least 1.0 g/day metformin or at least 1.5 g/day metformin for 3 months. In the present invention, a subject the diabetes type 2 of which is not adequately controlled may have a HbA1c value in the range of 7 % to 10%.

Yet another aspect of the present invention is the use of a combination comprising
(a) desPro³⁶Exendin-4(1-39)-Lys₆-NH₂ or/and a pharmaceutically acceptable salt thereof, and
(b) metformin or/and a pharmaceutically acceptable salt thereof,
for the production of a medicament for the treatment of diabetes mellitus type 2, wherein the subject to be treated is obese and has a body mass index of at least 30 kg/m² and has an age in the range of 18 to 50 years, and wherein the diabetes mellitus type 2 is not adequately controlled with metformin alone, and wherein desPro³⁶Exendin-4(1-39)-Lys₆-NH₂ or/and the pharmaceutically acceptable salt thereof is prepared for administration by one injection per day.

The medicament comprises desPro³⁶Exendin-4(1-39)-Lys₆-NH₂ and metformin in separate formulations, as described herein.

The invention is further illustrated by the following example.

### Example

### 24-week study comparing lixisenatide (AVE0010) to sitagliptin as add-on to metformin in obese type 2 diabetic patients younger than 50

Subject of the example is a randomized, double-blind, double-dummy, 2-arm parallel-group, multicenter, 24-week study comparing the efficacy and safety of lixisenatide (AVE0010) to sitagliptin (CAS Number 486460-32-6) as add-on to metformin in obese type 2 diabetic patients younger than 50 years and not adequately controlled with metformin. Sitagliptin is an antidiabetic drug, acting as an inhibitor of dipeptidyl peptidase 4 (DPP4) resulting in enhanced level of Glucagon-Like Peptide 1, thereby reducing blood glucose levels in diabetic patients.

### Study Primary Objectives

The primary objective of this study is to assess the efficacy of lixisenatide on a composite endpoint of glycemic control (HbA1c) and body weight in comparison to sitagliptin as an add-on treatment to metformin over a period of 24 weeks in obese type 2 diabetic patients younger than 50.

Study Secondary Objectives are assessment of the effects of lixisenatide on:
- Absolute changes in HbA1c and body weight
- Fasting plasma glucose
- Plasma glucose, insulin, C peptide, glucagon and proinsulin during a 2-hour standardized meal test
- Insulin resistance assessed by HOMA-IR
- Beta cell function assessed by HOMA-beta
- To assess lixisenatide safety and tolerability
- To assess lixisenatide PK using the population PK approach and to assess anti-lixisenatide antibody development.

### Specific vulnerable populations:

Women of child-bearing potential using contraception.

### Inclusion criteria

Patients (male and female) with type 2 diabetes mellitus, as defined by WHO (21), diagnosed for at least 1 year at the time of screening visit, insufficiently controlled with metformin at a stable dose of at least 1.5 g/day, for at least 3 months prior to the screening visit. Patients with obesity (BMI ≥ 30kg/m²) and aged from 18 years to less than 50 years.

### Exclusion criteria

- HbA1 c <7.0% or HbA1 c >10% at screening
- Type 1 diabetes mellitus
- Pregnancy or lactation
- Women of childbearing potential with no effective contraceptive method Fasting Plasma Glucose at screening >250 mg/dL (>13.9 mmol/L)
- Weight change of more than 5 kg during the 3 months preceding the screening visit
- History of unexplained pancreatitis, chronic pancreatitis, pancreatectomy, stomach/gastric surgery, inflammatory bowel disease
- History of metabolic acidosis, including diabetic ketoacidosis within 1 year prior to screening
- Hemoglobinopathy or hemolytic anemia or receipt of blood or plasma products within 3 months prior to the time of screening
- Within the last 6 months prior to screening: history of myocardial infarction, stroke, or heart failure requiring hospitalization
- Known history of drug or alcohol abuse within 6 months prior to the time of screening
- Any clinically significant abnormality identified on physical examination, laboratory tests, ECG or vital signs at the time of screening that in the judgment of the investigator or any sub investigator would preclude safe completion of the study or constrains efficacy assessment such as major systemic diseases, presence of clinically significant diabetic retinopathy or presence of macular edema likely to require laser treatment within the study period
- Uncontrolled or inadequately controlled hypertension at the time of screening with a resting systolic or diastolic blood pressure >180 mmHg or >110 mmHg, respectively
- Laboratory findings at the time of screening:
   - Amylase and/or lipase >3 times the upper limit of the normal laboratory range
   - Total bilirubin: >1.5 times the upper limit of the normal laboratory range (except in case of Gilbert's syndrome)
   - Hemoglobin <11 g/dL and/or neutrophils <1,500/mm³ and/or platelets <100,000/mm³
   - Positive test for Hepatitis B surface antigen and/or Hepatitis C antibody
   - Positive serum pregnancy test in females of childbearing potential
- Use of other oral or injectable antidiabetic or hypoglycemic agents than metformin (e.g., sulfonylurea, alpha glucosidase inhibitor, thiazolidinedione, exenatide, DPP-IV inhibitors, insulin etc.) within 3 months prior to the time of screening
- Unstable diet or unstable anti-obesity treatment within 3 months prior to the time of screening
- Use of systemic glucocorticoids (excluding topical application or inhaled forms) for one week or more within 3 months prior to the time of screening
- Use of any investigational drug within 3 months prior to screening
- Clinically relevant history of gastrointestinal disease associated with prolonged nausea and vomiting, including, but not limited to gastroparesis and gastroesophageal reflux disease requiring medical treatment, within 6 months prior to the time of screening
- Any previous treatment with lixisenatide (e.g. participation in a previous study with lixisenatide)
- Allergic reaction to any GLP 1-agonist in the past (e.g. exenatide, liraglutide) or to metacresol
- History of a serious hypersensitivity reaction to sitagliptin.
- Moderate or severe renal impairment (creatinine clearance inferior to 50 ml/min)

### Duration of study period per subject

Maximum duration of 27 weeks ± 7 days (3-week screening + 24-week double-blind, double-dummy, active-controlled treatment + 3-day follow-up)

### INVESTIGATIONAL PRODUCTS

| INN | Compound code | Pharmaceutical form | Route of administration |
|---|---|---|---|
| Lixisenatide | AVE0010 | injection | subcutaneous |
| Sitagl iptin | | capsules | capsules |

### STUDY ARMS Number of arms: 2

| Arm Label | Arm description | Arm type |
|---|---|---|
| Lixisenatide | Injection of lixisenatide once a day in the morning within 1 hour prior to breakfast (first 2 weeks of double-blind period: titration 10 to 15 µg, then 15 to 20 µg) and one capsule of sitagliptin placebo intake in the morning or without food. On top of metformin background therapy. | Experimental |
| Sitagliptin | One capsule of sitagliptin intake in the morning with or without food and lixisenatide matched placebo injection once a day in the morning within hour prior to breakfast. On top of metformin background therapy. | Active Calibrator/Comparator |

### ENDPOINTS

| Primary Endpoint(s): | Time frame for evaluation |
|---|---|
| Percentage of patients with HbA1c values <7% AND a weight loss of at least 5% of baseline body weight | 24 weeks |
| | |
| Secondary Endpoint(s): | Time frame for evaluation |
| Absolute change in HbA1c values | 24 weeks |
| Percentage of patients with HbA1 c values ≤ 6.5% | 24 weeks |
| Absolute change in body weight | 24 weeks |
| Change in fasting plasma glucose | 24 weeks |
| Change in plasma glucose and in β-cell function during a test meal | 24 weeks |
| Change in insulin resistance assessed by HOMA-IR | 24 weeks |
| Change in β-cell function assessed by HOMO-3 | 24 weeks |
| Percentage of patients requiring rescue therapy during the double-blind period | 24 weeks |

## Claims

1. A combination for use in the treatment of diabetes mellitus type 2, the combination comprising
(a) desPro³⁶Exendin-4(1-39)-Lys₆-NH₂ or/and a pharmaceutically acceptable salt thereof,
(b) metformin or/and a pharmaceutically acceptable salt thereof, wherein the subject to be treated is obese and has a body mass index of at least 30 kg/m² and has an age in the range of 18 to 50 years, and wherein the diabetes mellitus type 2 is not adequately controlled with metformin alone, and wherein desPro³⁶Exendin-4(1-39)-Lys₆-NH₂ or/and the pharmaceutically acceptable salt thereof is prepared for administration by one injection per day.

2. The combination for use according to claim 1, wherein desPro³⁶Exendin-4(1-39)-Lys₆-NH₂ or/and a pharmaceutically acceptable salt thereof is administered subcutaneously.

3. The combination for use according to claim 1 or 2, wherein the metformin is administered orally.

4. The combination for use according to any one of the preceding claims, wherein desPro³⁶Exendin-4(1-39)-Lys₆-NH₂ or/and a pharmaceutically acceptable salt is administered in an add-on therapy to administration of metformin.

5. The combination for use according to any one of the preceding claims, wherein the subject to be treated is an adult subject.

6. The combination for use according to any one of the preceding claims, wherein treatment with a dose of at least 1.5 g/day metformin alone for three months does not adequately control diabetes mellitus type 2.

7. The combination for use according to any one of the preceding claims, wherein the subject to be treated has a HbA1c value in the range of 7% to 10%.

8. Use of a combination of
(a) desPro³⁶Exendin-4(1-39)-Lys₆-NH₂ or/and a pharmaceutically acceptable salt thereof,
(b) metformin or/and a pharmaceutically acceptable salt thereof,
for the production of a medicament for the treatment of diabetes mellitus type 2, wherein the subject to be treated is obese and has a body mass index of at least 30 kg/m² and has an age in the range of 18 to 50 years, and wherein the diabetes mellitus type 2 is not adequately controlled with metformin alone, and wherein desPro³⁶Exendin-4(1-39)-Lys₆-NH₂ or/and the pharmaceutically acceptable salt thereof is prepared for administration by one injection per day.

## Patentansprüche

1. Kombination zur Verwendung bei der Behandlung von Diabetes mellitus vom Typ 2, wobei die Kombination Folgendes umfasst:
(a) desPro³⁶Exendin-4 (1-39)-Lys₆-NH₂ und/oder ein pharmazeutisch unbedenkliches Salz davon,
(b) Metformin und/oder ein pharmazeutisch unbedenkliches Salz davon,
wobei der zu behandelnde Patient adipös ist und einen Körpermasseindex von mindestens 30 kg/m² und ein Alter im Bereich von 18 bis 50 Jahren hat und wobei der Diabetes mellitus vom Typ 2 durch Metformin alleine nicht ausreichend kontrolliert wird und wobei desPro³⁶Exendin-4 (1-39)-Lys₆-NH₂ und/oder das pharmazeutisch unbedenkliche Salz davon für eine Verabreichung durch eine Injektion pro Tag zubereitet ist.

2. Kombination zur Verwendung nach Anspruch 1, wobei desPro³⁶Exendin-4 (1-39)-Lys₆-NH₂ und/oder ein pharmazeutisch unbedenkliches Salz davon subkutan verabreicht wird.

3. Kombination zur Verwendung nach Anspruch 1 oder 2, wobei das Metformin oral verabreicht wird.

4. Kombination zur Verwendung nach einem der vorhergehenden Ansprüche, wobei desPro³⁶Exendin-4(1-39)-Lys₆-NH₂ und/oder ein pharmazeutisch unbedenkliches Salz davon als Add-on-Therapie zur Verabreichung von Metformin verabreicht wird.

5. Kombination zur Verwendung nach einem der vorhergehenden Ansprüche, wobei es sich bei dem zu behandelnden Patienten um einen erwachsenen Patienten handelt.

6. Kombination zur Verwendung nach einem der vorhergehenden Ansprüche, wobei eine Behandlung mit einer Dosis von mindestens 1,5 g/Tag Metformin alleine über drei Monate Diabetes mellitus vom Typ 2 nicht ausreichend kontrolliert.

7. Kombination zur Verwendung nach einem der vorhergehenden Ansprüche, wobei der zu behandelnde Patient einen HbA1c-Wert im Bereich von 7% bis 10% hat.

8. Verwendung einer Kombination von
(a) desPro³⁶Exendin-4(1-39)-Lys₆-NH₂ und/oder ein pharmazeutisch unbedenkliches Salz davon,
(b) Metformin und/oder ein pharmazeutisch unbedenkliches Salz davon,
zur Herstellung eines Medikaments zur Behandlung von Diabetes mellitus vom Typ 2, wobei der zu behandelnde Patient adipös ist und einen Körpermasseindex von mindestens 30 kg/m² und ein Alter im Bereich von 18 bis 50 Jahren hat und wobei der Diabetes mellitus vom Typ 2 durch Metformin alleine nicht ausreichend kontrolliert wird und wobei desPro³⁶Exendin-4 (1-39)-Lys₆-NH₂ und/oder das pharmazeutisch unbedenkliche Salz davon für eine Verabreichung durch eine Injektion pro Tag zubereitet ist.

## Revendications

1. Combinaison pour utilisation dans le traitement du diabète sucré de type 2, la combinaison comprenant
(a) de la desPro³⁶Exendine-4(1-39)-Lys₆-NH₂ et/ou l'un des sels pharmaceutiquement acceptables de celle-ci,
(b) de la metformine et/ou l'un des sels pharmaceutiquement acceptables de celle-ci,
où le sujet à traiter est obèse, présente un indice de masse corporelle d'au moins 30 kg/m² et est âgé d'entre 18 et 50 ans, où le diabète sucré de type 2 n'est pas correctement régulé par la metformine seule, et où la desPro³⁶Exendin-4(1-39)-Lys₆-NH₂ et/ou le sel plnarmaceutiquement acceptable de celle-ci sont préparés pour administration à raison d'une injection par jour.

2. Combinaison pour utilisation selon la revendication 1, où la desPro³⁶Exendin-4 (1-39)-Lys₆-NH₂ et/ou l'un des sels pharmaceutiquement acceptable de celle-ci sont administrés par voie sous-cutanée.

3. Combinaison pour utilisation selon la revendication 1 ou 2, où la metformine est administrée par voie orale.

4. Combinaison pour utilisation selon l'une quelconque des revendications précédentes, où la desPro³⁶Exendine-4(1-39)-Lys₆-NH₂ et/ou l'un des sels pharmaceutiquement acceptables de celle-ci sont administrés en tant que thérapie auxiliaire de l'administration de metformine.

5. Combinaison pour utilisation selon l'une quelconque des revendications précédentes,
où le sujet à traiter est un sujet adulte.

6. Combinaison pour utilisation selon l'une quelconque des revendications précédentes, où le traitement par une dose d'au moins 1,5 g/jour de metformine seule pendant trois mois ne régule pas correctement le diabète sucré de type 2.

7. Combinaison pour utilisation selon l'une quelconque des revendications précédentes, où le sujet à traiter présente une teneur en HbA1c comprise entre 7 % et 10 %.

8. Utilisation d'une combinaison de
(a) desPro³⁶Exendine-4(1-39)-Lys₆-NH₂ et/ou de l'un des sels pharmaceutiquement acceptables de celle-ci,
(b) metformine et/ou de l'un des sels pharmaceutiquement acceptables de celle-ci,
dans la production d'un médicament destiné au traitement du diabète sucré de type 2,
où le sujet à traiter est obèse, présente un indice de masse corporelle d'au moins 30 kg/m² et est âgé d'entre 18 et 50 ans, où le diabète sucré de type 2 n'est pas correctement régulé par la metformine seule, et où la desPr0³⁶Exendin-4(1-39)-Lys6-NH₂ et/ou le sel pharmaceutiquement acceptable de celle-ci sont préparés pour administration à raison d'une injection par jour.


### SEQUENCE LISTING

<110> Sanofi-Aventis Deutschland GmbH
<120> Method of treatment of diabetes type 2 comprising add-on therapy to metformin
<130> DE2009/199
<160> 2
<170> PatentIn version 3.3
<210> 1
   <211> 44
   <212> PRT
   <213> Artificial
<220>
   <223> AVE0010 (44 AS)
   Position 44 (Lys) is amidated
<400> 1
   His Gly Glu Gly Thr Phe Thr Ser Asp Leu Ser Lys Gln Met Glu Glu
   1               5                   10                  15
   Glu Ala Val Arg Leu Phe Leu Glu Trp Leu Lys Asn Gly Gly Pro Ser
               20                  25                  30
   Ser Gly Ala Pro Pro Ser Lys Lys Lys Lys Lys Lys
           35                  40
<210> 2
   <211> 39
   <212> PRT
   <213> Artificial
<220>
   <223> Heloderma Suspectum
   Position 39 (Ser) is amidated
<400> 2
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35